(19) Europäisches Patentamt
European Patent Office
Office européen des brevets

(11) **EP 3 905 716 B1**

(12) **EUROPEAN PATENT SPECIFICATION**

(45) Date of publication and mention
of the grant of the patent:
**04.10.2023  Bulletin 2023/40**

(21) Application number: **19901949.8**

(22) Date of filing: **13.12.2019**

(51) International Patent Classification (IPC):
*H04R 17/00* (2006.01)   *H04R 19/04* (2006.01)
*A61B 8/14* (2006.01)   *A61B 8/02* (2006.01)

(52) Cooperative Patent Classification (CPC):
**H04R 17/005; H04R 19/04;** A61B 8/02; A61B 8/14;
H04R 2201/003

(86) International application number:
**PCT/JP2019/048874**

(87) International publication number:
**WO 2020/137601 (02.07.2020 Gazette 2020/27)**

(54) **ULTRASOUND DEVICE**

ULTRASCHALLVORRICHTUNG

DISPOSITIF À ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **26.12.2018   JP 2018242884**

(43) Date of publication of application:
**03.11.2021   Bulletin 2021/44**

(73) Proprietor: **Sony Group Corporation**
**Tokyo 108-0075 (JP)**

(72) Inventor: **MORIMOTO, Rui**
**Tokyo 108-0075 (JP)**

(74) Representative: **D Young & Co LLP**
**120 Holborn**
**London EC1N 2DY (GB)**

(56) References cited:
WO-A1-2018/139194     DE-T5-112012 002 438
JP-A- 2011 071 842     JP-A- 2013 005 040
JP-A- 2018 502 467

**Description**

Technical Field

The present technology relates to an ultrasonic apparatus for transmitting and receiving a ultrasonic wave.

Background Art

Since an array type ultrasonic apparatus capable of transmitting and receiving an ultrasonic wave can acquire information about a biological structure, a heart rate, a blood flow, and the like, applications to not only a medical care but also general consumer products such as healthcare devices are considered.

The ultrasonic apparatus is generally formed by piezoelectric elements which produce an ultrasonic vibration by applying a voltage. However, in a conventional structure, a size of an acoustic absorbing layer called a backing is large, and although there is no problem in general medical care applications, the size is too large to be mounted on a wearable device.

In addition, the array type ultrasonic apparatus is generally manufactured by dicing called Dice & Fill and filling kerf grooves with epoxy resin, but this manufacturing method is poor in throughputs because dicing is performed for each array. Furthermore, since wiring is drawn from electrodes, it is necessary to perform a bonding process on a land of a flexible substrate by individual ultrasonic apparatus, which results in a problem in mass productivity.

On the other hand, the array type ultrasonic apparatus which adopts an MEMS (Micro Electro Mechanical Systems) structure is noticed in recent years. The use of the MEMS enables the apparatus to be miniaturized and reduced in height, and its application to an intravascular ultrasonic catheter and the like is also studied. For example, Patent Literature 1 discloses an ultrasonic probe having the MEMS structure.

In the MEMS, voids are provided in a piezoelectric material, and a beam structure called a membrane structure is formed. By receiving a charge in a form of current generated by oscillating the membrane structure, detection of ultrasonic wave is possible. Since the MEMS can be manufactured in a semiconductor process, the mass productivity is high. Since an amplifier circuit or the like can be mounted on the same substrate, an S/N ratio of a received signal is also high.

Patent Literature 2 describes an ultrasonic probe and method for manufacturing an ultrasonic transducer array, and Patent Literature 3 describes another example of an ultrasonic sensor device.

Citation List

Patent Literature

    Patent Literature 1: Japanese Patent Application Laid-open No. 2011-071842
    Patent Literature 2: JP 2011 071842 A
    Patent Literature 3: DE 2012 002438 T5

Disclosure of Invention

Technical Problem

However, in the MEMS based on the membrane structure, it is difficult to generate an ultrasonic wave having a high transmission sound pressure, and reliability is also insufficient.

In view of the above circumstances, an object of the present technology is to provide an ultrasonic apparatus having a high transmission strength, high reception sensitivity, reliability, and excellent mass productivity.

Solution to Problem

The invention is defined by the claims.

According to the invention, since the ultrasonic apparatus has the MEMS structure, and includes the ultrasonic wave receiving element having high reception sensitivity and the ultrasonic wave transmitting element having a high ultrasonic transmission strength, the ultrasonic apparatus has a high transmission strength and high reception sensitivity. Furthermore, the ultrasonic apparatus can be manufactured by a semiconductor process, it can be made to be excellent in reliability and mass productivity.

At least one of the upper electrode and the lower electrode may be an array electrode for independently controlling the ultrasonic wave transmitting element as an array element.

The piezoelectric material layer between the array electrodes may not be provided with a groove structure.

The ultrasonic apparatus may further include an acoustic absorbing layer made of an acoustic absorbing material and acoustically bonded to the ultrasonic wave transmitting element.

The acoustic absorbing layer may have an acoustic impedance equal to or less than 1/10 of an acoustic impedance of the piezoelectric material layer.

The ultrasonic apparatus may further include an acoustic matching layer provided on the ultrasonic wave receiving element on a side opposite to the ultrasonic wave transmitting element.

The ultrasonic wave receiving element may have a piezoelectric material thin film.

The piezoelectric material thin film may be a thin film having a thickness thinner than the piezoelectric material layer.

The piezoelectric material thin film may be made of an inorganic piezoelectric material, an organic piezoelectric material, or a combination thereof.

The ultrasonic apparatus may further include a semiconductor substrate arranged between the ultrasonic wave transmitting element and the ultrasonic wave receiving element.

In the semiconductor substrate, a cavity may be provided between the piezoelectric material thin film and the ultrasonic wave transmitting element.

A semiconductor circuit is provided on a piezoelectric material layer side of the semiconductor substrate.

The semiconductor circuit may include one or more of a charge pumping circuit, a driver circuit, an address selection circuit, an amplifier circuit, an analog arithmetic circuit, an analog-to-digital conversion circuit, a digital arithmetic circuit, a power supply circuit, a modulation circuit, and an impedance matching circuit.

The amplifier circuit may be any of a low noise circuit, a differential amplifier, and a logarithmic conversion amplifier.

The semiconductor substrate may have a through electrode for connecting the ultrasonic wave receiving element and the semiconductor circuit.

The piezoelectric material layer may have a through via electrode for connecting the semiconductor circuit and the lower electrode.

Brief Description of Drawings

[Fig. 1] Fig. 1 is a perspective diagram of an ultrasonic apparatus according to an embodiment of the present technology.

[Fig. 2] Fig. 2 is a perspective diagram of the ultrasonic apparatus seen from an opposite side.

[Fig. 3] Fig. 3 is a cross-sectional diagram of the ultrasonic apparatus.

[Fig. 4] Fig. 4 is a cross-sectional diagram of an array region included in the ultrasonic apparatus.

[Fig. 5] Fig. 5 is a cross-sectional diagram of a part of the array region included in the ultrasonic apparatus.

[Fig. 6] Fig. 6 is a schematic diagram showing a manufacturing process of the ultrasonic apparatus.

[Fig. 7] Fig. 7 is a schematic diagram showing a manufacturing process of the ultrasonic apparatus.

[Fig. 8] Fig. 8 is a schematic diagram showing a manufacturing process of the ultrasonic apparatus.

[Fig. 9] Fig. 9 is a schematic diagram showing a manufacturing process of the ultrasonic apparatus.

[Fig. 10] Fig. 10 is a schematic diagram showing a manufacturing process of the ultrasonic apparatus.

[Fig. 11] Fig. 11 is a schematic diagram showing a manufacturing process of the ultrasonic apparatus.

[Fig. 12] Fig. 12 is a schematic diagram showing a manufacturing process of the ultrasonic apparatus.

[Fig. 13] Fig. 13 is a schematic diagram showing a manufacturing process of the ultrasonic apparatus.

[Fig. 14] Fig. 14 is a schematic diagram showing a manufacturing process of the ultrasonic apparatus. Mode(s) for Carrying Out the Invention

An ultrasonic apparatus according to an embodiment of the present technology will be described.

[Overall Configuration of Ultrasonic Apparatus]

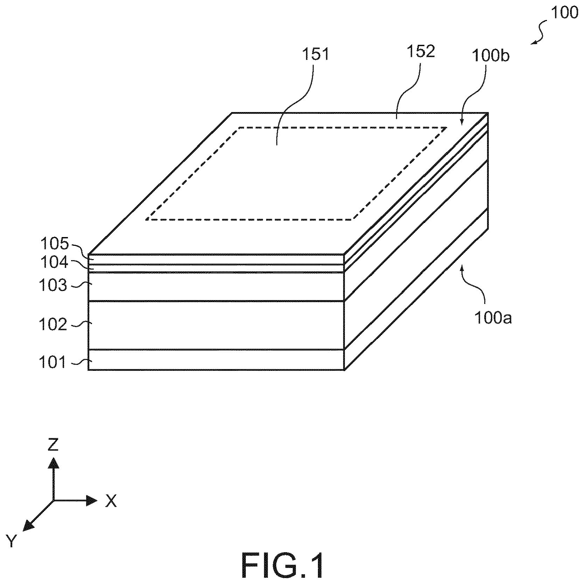

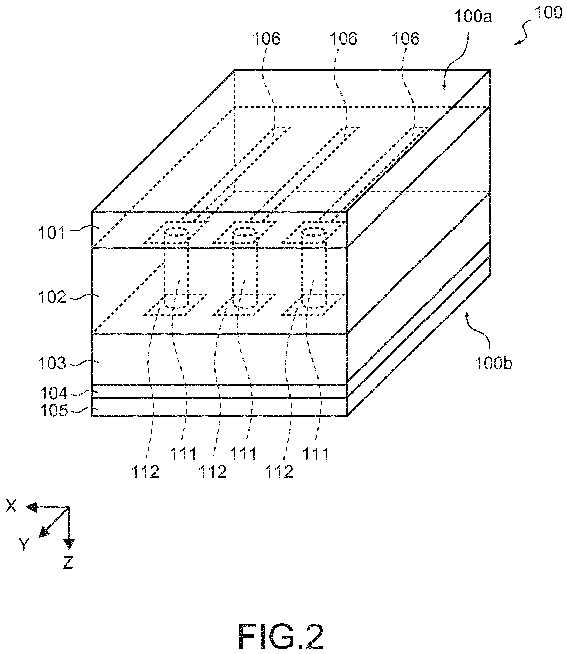

Fig. 1 is a perspective diagram of an ultrasonic apparatus 100 according to the present embodiment. Fig. 2 is a diagram of the ultrasonic apparatus 100 seen from a back side of Fig. 1 and is a perspective diagram showing parts perspective. In the following drawings, three mutually orthogonal directions are referred to as the X direction, the Y direction, and the Z direction, respectively.

The ultrasonic apparatus 100 is an apparatus capable of transmitting and receiving an ultrasonic wave. The ultrasonic apparatus 100 can acquire information of a target to be measured by transmitting the ultrasonic wave to the target to be measured and receiving a reflected wave of the target to be measured. The target to be measured is typically a living body, and the ultrasonic apparatus 100 is available in various fields such as medical use and health care. In addition, the target to be measured is not limited to the living body, and the ultrasonic apparatus 100 can be used in various fields in which a measurement by the ultrasonic wave is possible.

As shown in Fig. 1, the ultrasonic apparatus 100 has an array region 151 and a peripheral circuit region 152. The array region 151 is provided in a center of the ultrasonic apparatus 100 and is a region in which an array of ultrasonic elements are formed. The peripheral circuit region 152 is provided around the array region 151, and a region in which circuits relating to transmission and reception of the ultrasonic wave or the like are provided.

Note that an arrangement of the array region 151 and the peripheral circuit region 152 is not limited to that shown in Fig. 1, and, for example, the peripheral circuit region 152 may be provided only on one side of the ultrasonic apparatus 100.

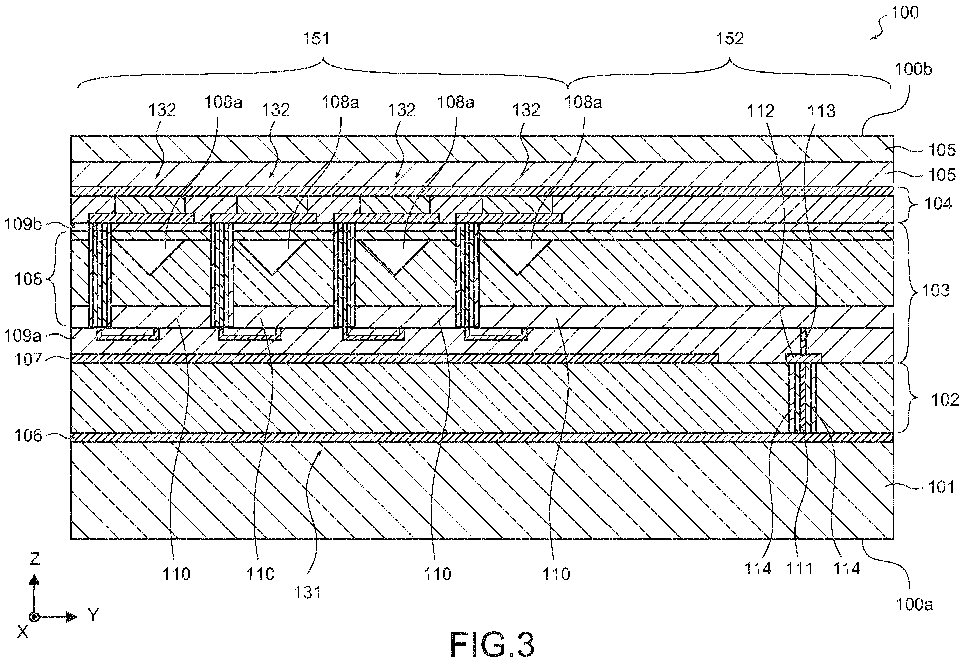

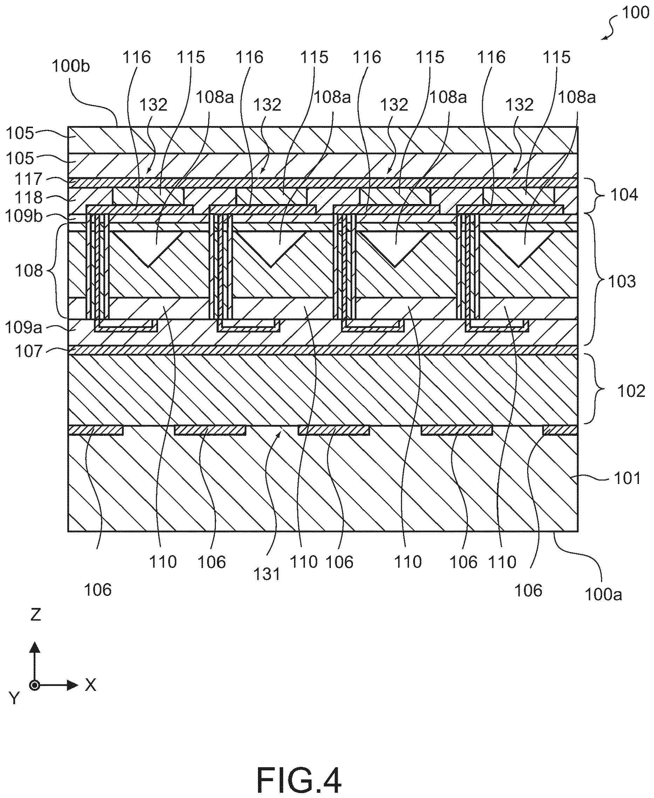

Fig. 3 is a cross-sectional diagram in the Y-Z plane of the ultrasonic apparatus 100 including the array region 151 and the peripheral circuit region 152, and Fig. 4 is a cross-sectional diagram of the ultrasonic apparatus 100 including only the array region 151 in the X-Z plane. As shown in Figs. 3 and 4, the ultrasonic apparatus 100 includes an acoustic absorbing layer 101, a piezoelectric material layer 102, a semiconductor substrate 103, a MEMS layer 104, an acoustic matching layer 105, lower electrodes 106, and an upper electrode 107.

The array region 151 includes an ultrasonic wave transmitting element 131 and ultrasonic wave receiving elements 132 to be described later, and the peripheral circuit region 152 includes no ultrasonic wave transmitting element 131 and no ultrasonic wave receiving element 132 and includes a peripheral circuit.

The ultrasonic apparatus 100 is arranged so that the acoustic matching layer 105 contacts the target to be measured such as a living body. Of the front and back surfaces of the ultrasonic apparatus 100, a surface on the side of the acoustic absorbing layer 101 is defined as a first surface 100a, and a surface on the side of the acoustic matching layer 105, which is the opposite side of the first surface 100a, is defined as a second surface 100b.

The ultrasonic wave transmitting element 131 is formed of the piezoelectric material layer 102, the lower electrodes 106, and the upper electrode 107, and is arranged on a first surface 100a side of the ultrasonic apparatus 100. The ultrasonic wave receiving elements 132 are formed of the MEMS layer 104, and are arranged between the second surface 100b and the ultrasonic wave transmitting element 131.

The ultrasonic wave transmitting element 131 transmits the ultrasonic wave toward a second surface 100b side. The reflected wave of the ultrasonic wave generated by the target to be measured is received by the ultrasonic wave receiving elements 132.

The acoustic absorbing layer 101 is acoustically bonded to the piezoelectric material layer 102 to absorb a reflected acoustic wave accumulated in the piezoelectric material layer 102 and reduce reverberation. The acoustic absorbing layer 101 may be made of an acoustic absorbing material such as a material obtained by mixing a filler and a synthetic resin. The acoustic absorbing layer 101 desirably has an acoustic impedance equal to or less than 1/10 of the acoustic impedance of the piezoelectric material layer 102. In addition, the acoustic absorbing layer 101 may also take a de-matching structure that reflects an unwanted acoustic wave toward the piezoelectric material layer 102 and adds them to a transmitted acoustic wave to enhance the transmitted acoustic waves.

The piezoelectric material layer 102 is arranged on the acoustic absorbing layer 101 and is a layer that generates an ultrasonic vibration in the ultrasonic wave transmitting element 131. The piezoelectric material layer 102 may be made of an inorganic piezoelectric material, an organic piezoelectric material, or a combination thereof.

For example, the piezoelectric material layer 102 may be made of PZT (lead zirconate titanate). The piezoelectric material layer 102 may be made of, in addition to the PZT, a lead-based piezoelectric material such as PMN-PT (lead magnesium niobate - lead titanate), a lead-free piezoelectric material such as BTO (barium titanate), AlN, and BZT-BCT (barium zirconate-titanate/barium calcium-titanate), or an organic piezoelectric material such as PVDF (polyvinylidene fluoride), PVDT-TrFE (vinylidene fluoride/trifluoride copolymer), a polyamino acid material, and a polylactic acid material.

In a case where the semiconductor substrate 103 is made of silicon, the piezoelectric material layer 102 is desirably made of a piezoelectric material having an acoustic impedance relatively close to the acoustic impedance of the semiconductor substrate 103, an organic-inorganic composite material such as PZT (to 30 MRayls), PMN-PT (34 MRayls), BTO (to 30 MRayls), AlN (36 MRayls) and a 1-3 composite, because the acoustic impedance of the semiconductor substrate 103 acoustically connected is 19.7 MRayls.

Each lower electrode 106 of the ultrasonic wave transmitting element 131 is provided between the piezoelectric material layer 102 and the acoustic absorbing layer 101. The lower electrodes 106, as shown in Figs. 2 and 4, are an array electrode provided in an array, and extends from the peripheral circuit region 152 to the array region 151. Each lower electrode 106 is made of a conductive material such as Ag, Ni, Ti, Au and Cu.

The upper electrode 107 of the ultrasonic wave transmitting element 131 is provided between the piezoelectric material layer 102 and the semiconductor substrate 103. The upper electrode 107, as shown in Figs. 3 and 4, is provided in a planar shape in the array region 151. The upper electrode 107 is made of a conductive material such as Ag, Ni, Ti, Au and Cu.

Note that the upper electrodes 107 may be the array electrode, and the lower electrode 106 may be formed in the planar shape.

In the array region 151, the piezoelectric material layer 102 is sandwiched between each lower electrode 106 and the upper electrode 107, and the ultrasonic wave transmitting element 131 is configured.

The semiconductor substrate 103 is a substrate made of a semiconductor material, is arranged between the piezoelectric material layer 102 and the MEMS layer 104, and is sandwiched between the ultrasonic wave transmitting element 131 and the ultrasonic wave receiving elements 132 in the array region 151. The semiconductor substrate 103 includes a semiconductor 108, an insulator 109a, and an insulator 109b. The insulator 109a is provided on a piezoelectric layer 102 side of the semiconductor substrate 103, and the insulator 109b is provided on a MEMS layer 104 side of the semiconductor substrate 103. The semiconductor 108 is provided between the insulator 109a and the insulator 109b.

The insulators 109a and 109b may be parts formed by oxidizing the material of the semiconductor 108, the semiconductor 108 may be made of, for example, silicon, and the insulators 109a and 109b may be made of, for example, silicon oxide ($SiO_2$).

In the semiconductor 108, cavities 108a, which is a gap, is provided on the first surface 100a side of the ultrasonic wave receiving elements 132. Furthermore, a semiconductor circuit 110 is provided on the piezoelectric material layer 102 side in the semiconductor 108.

The semiconductor circuit 110 is a circuit formed by doping p-type and n-type impurities in the semiconductor 108. The details of the semiconductor circuit 110 will be described later.

The MEMS layer 104 is arranged on the semiconductor substrate 103 and is constituted by a MEMS (Micro Electro Mechanical Systems). The MEMS layers 104 form ultrasonic wave receiving elements 132.

The acoustic matching layer 105 is arranged on the MEMS layer 104 to reduce a difference in the acoustic impedance of the target to be measured and of the ultrasonic wave receiving elements 132 and to prevent reflection of the ultrasonic wave to the target to be measured. The acoustic matching layer 105 increases the transparency and energy efficiency of the ultrasonic wave at the time of transmission and reception, and suppresses an effect of the reverberation.

The acoustic matching layer 105 may have two layers, and the acoustic impedance of the uppermost layer is desirably 1.5 to 4 MRayls and the acoustic impedance of the second layer is desirably 3 to 8 MRayls when the target to be measured is the living body. The acoustic matching layer 105 may be provided with one layer or three or more layers. The material of the acoustic matching layer 105 may be a synthetic resin or a ceramic material.

As shown in Figs. 2 and 3, the piezoelectric material layer 102 in the peripheral circuit region 152 is provided with through via electrodes 111 piercing through the piezoelectric material layer 102. The through via electrodes 111 connect the lower electrodes 106 and electrode pads 112 and are connected to the semiconductor circuit 110 via the wiring 113. Around the through via electrodes 111, the insulators 114 are provided to insulate the through via electrodes 111 and the piezoelectric material layer 102.

Thus, a driving signal generated in the semiconductor circuit 110 is supplied to the lower electrodes 106 via the through via electrodes 111, the ultrasonic wave transmitting element 131 is driven.

[Structure of Array Portion]

As described above, the ultrasonic wave transmitting element 131 is formed of the piezoelectric material layer 102, the lower electrodes 106, and the upper electrode 107. The piezoelectric material layer 102 is sandwiched between each lower electrode 106 and the upper electrode 107, and when a voltage is applied between the lower electrodes 106 and the upper electrode 107, generates vibration due to a reverse piezoelectric effect, and generates the ultrasonic wave.

The upper electrode 107 is formed in the planar shape on the piezoelectric material layer 102 and is connected to ground. The lower electrodes 106 have an array structure as shown in Fig. 4. Thus, by phase-controlling a potential of each lower electrode 106 independently, it is possible to transmit beamforming.

Note that a thickness of the piezoelectric material layer 102 is set to oscillate at a frequency determined by calculation using a frequency constant of the piezoelectric material. A calculation formula is expressed by the following (Expression 1).

$$N = f_r \times t \quad \text{(Expression 1)}$$

In (Expression 1), N is a frequency constant, $f_r$ is a resonant frequency, and t is a thickness of the piezoelectric layer 102. If the resonant frequency is 7 MHz, t is about 150 $\mu$m.

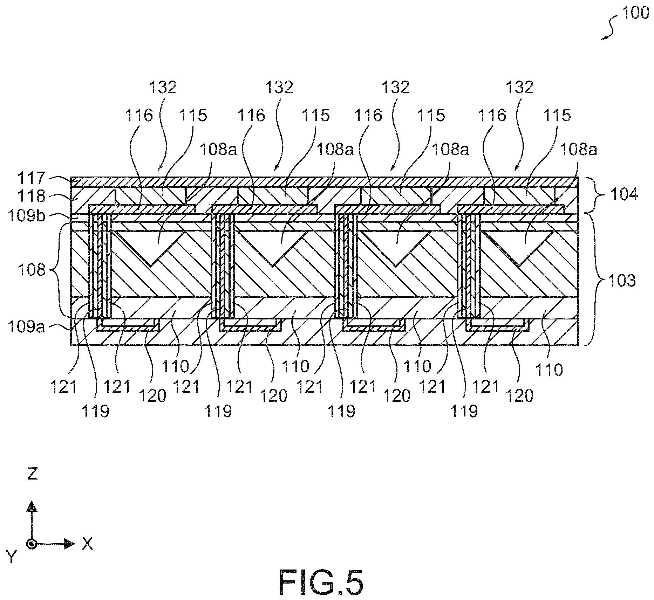

The ultrasonic wave receiving elements 132 are provided in the MEMS layers 104 as described above. Fig. 5 is a cross-sectional diagram showing only the semiconductor substrate 103 and the MEMS layer 104 of the ultrasonic apparatus 100 and is a part of Fig. 4. As shown in Fig. 5, the MEMS layer 104 includes piezoelectric material thin films 115, lower electrode films 116, an upper electrode film 117, and an interlayer insulating film 118.

Each piezoelectric material thin film 115 is sandwiched between each lower electrode film 116 and the upper electrode film 117 to form the ultrasonic wave receiving elements 132. A plurality of ultrasonic wave receiving elements 132 is provided to form an array.

The piezoelectric material thin films 115 are thin films that receive the ultrasonic wave in the ultrasonic wave receiving elements 132, and a plurality of thin films is provided with the interlayer insulating film 118 interposed therebetween. The piezoelectric material thin films 115 may be each made of an inorganic piezoelectric material, an organic piezoelectric material, or a combination thereof.

Examples of the inorganic piezoelectric material include a lead-based piezoelectric material such as PZT, PMN-PT (lead magnesium niobate - lead titanate), and a non-lead-based piezoelectric material such as BTO (barium titanate), AIN, and BZT-BCT (barium calcium barium zirconate titanate).

Examples of the organic piezoelectric material include PVDF (polyvinylidene fluoride), PVDT-TrFE (vinylidene fluoride/ethylene trifluoride copolymer), a polyamino acid material, and a polylactic acid material.

A plurality of the lower electrode films 116 is arranged on the semiconductor substrate 103 for each piezoelectric material thin film 115. Each lower electrode film 116 functions as a signal extraction electrode of the ultrasonic wave receiving elements 132.

The upper electrode film 117 is arranged in a planar shape on the piezoelectric material thin films 115 and the interlayer insulating film 118. The upper electrode membrane 117 serves as ground.

The lower electrode films 116 and the upper electrode film 117 are made of a conductive material such as Pt. If the piezoelectric material thin films 115 are made of PZT, the lower electrode films 116 and the upper electrode film 117 are desirably Pt, so that an interface with the PZT is stabilized. Furthermore, the same effect can be obtained not only by Pt but also by ITO (indium tin oxide) or the like.

The interlayer insulating film 118 insulates the piezoelectric material thin films 115, the lower electrode films 116, and the upper electrode film 117. The interlayer insulating film 118 is made of an insulating material such as BPSG (Boron Phosphorus Silicon Glass).

In the ultrasonic wave receiving elements 132, when the reflected wave reflected by the target to be measured enters and the piezoelectric material thin films 115 vibrate, a potential difference is generated between each lower electrode film 116 and the upper electrode film 117 by the piezoelectric effect.

The cavities 108a are provided at a position immediately below the ultrasonic wave receiving elements 132 and between the piezoelectric material thin films 115 and the ultrasonic wave transmitting element 131. This cavity structure prevents the effect of the reverberation due to transmitted waves from the ultrasonic wave transmitting element 131 and reduces the acoustic impedance of the acoustic matching layer 105 to as relatively low as 1.5 to 7 MRayls when the living body is to be measured. Due to the cavity structure, the ultrasonic wave receiving elements 132 become a beam structure, causing flexure in the piezoelectric material thin films 115 and reducing effective acoustic impedance, which tends to cause acoustic matching.

The ultrasonic wave receiving elements 132 may each have a capacitive ultrasonic MEMS (cMUT) structure as shown in Fig. 5. Furthermore, the ultrasonic wave receiving elements 132 may each have a piezoelectric ultrasonic MEMS (pMUT) structure.

The cMUT requires a large bias to be applied to make it ready for reception, but the pMUT does not require this and can simplify the driver circuit.

The thickness of the piezoelectric material thin films 115 can be determined by the frequency constant of the piezoelectric material using the above (Expression 1) if the cavity structure is not provided. On the other hand, by providing the cavities 108a, the beam structure is formed, and rigidity of each portion between the piezoelectric material thin films 115 and the cavities 108a is reduced, so that a desired resonance frequency can be obtained with a thinner thickness.

Specifically, the ultrasonic wave receiving elements 132 can be regarded as diaphragm-type vibrators having the piezoelectric material thin films 115 as elastic thin films (diaphragms). The resonant frequency of the diaphragm-type vibrators is expressed by the following (Expression 2).

[Math. 1]

$$f_r = 0.5 \frac{t}{a^2} \sqrt{\frac{E}{\rho \left(1 + 0.669 \frac{\rho_{H_2O}}{\rho} \frac{a}{t}\right)}} \qquad \text{(Expression 2)}$$

In (Expression 2), t represents a diaphragm thickness, a represents a diaphragm radius, ρ represents a diaphragm density, E represents a diaphragm Young's modulus, and $\rho_{H2O}$ represents a water-load density.

For example, if the piezoelectric material thin films 115 are made of PZT, in order to produce the ultrasonic wave receiving elements 132 of 7 MHz, a = 60 μm, t = 20 um may be made thinner than the piezoelectric material layer 102 of the ultrasonic wave transmitting element 131 even at the same resonance frequency.

Furthermore, as shown in Fig. 5, the semiconductor substrate 103 is provided with through electrodes 119 piercing through the semiconductor substrate 103. The through electrodes 119 are connected to the lower electrode films 116 of the ultrasonic wave receiving elements 132 and are connected to the semiconductor circuit 110 via the wiring 120. Around the through electrodes 119, insulators 121 are provided to insulate the through electrodes 119 and the semiconductor substrate 103.

Thus, received signals generated in the ultrasonic wave receiving elements 132 are supplied to the semiconductor circuit 110 via the through electrodes 119, and signal processing is performed by the semiconductor circuit 110. The signal processing by the semiconductor circuit 110 includes analog signal processing such as amplifying, phasing addition, and detection, digital signal processing such as analog-to-digital conversion and FFT (fast Fourier transform), a part or all of data processing such as image processing.

[Operation and effect of ultrasonic apparatus]

The ultrasonic apparatus 100 has the above-described structure, and the ultrasonic wave transmitting element 131 is formed of a laminated structure on the first surface 100a side, and each ultrasonic wave receiving element 132 is formed of the MEMS on the second surface 100b side (see Fig. 4).

When the driving signal is supplied from the semiconductor circuit 110 to the lower electrodes 106 in the ultrasonic wave transmitting element 131, a potential difference between the lower electrodes 106 and the upper electrode 107 causes the ultrasonic vibration in the piezoelectric material layer 102, and the ultrasonic wave (transmitted wave) is generated. The ultrasonic wave travels to the second surface 100b side (upper side in Fig. 4) and is transmitted from the first surface 100a to the target to be measured.

In the ultrasonic wave transmitting element 131, by making the lower electrodes 106 to be the array structure, the potential of each lower electrode 106 is phase-controlled independently for each array, and it is possible to transmit beamforming.

The ultrasonic wave is reflected by the target to be measured and passes through the second surface 100b to reach the ultrasonic wave receiving elements 132. In the ultrasonic wave receiving elements 132, the piezoelectric material thin films 115 are vibrated by receiving the ultrasonic wave (received wave), and the received signals are output to the semiconductor circuit 110 via the lower electrode films 116.

Note that when seen from the vertical direction (Z direction) of the second surface 100b, the lower electrodes 106 of the ultrasonic wave transmitting element 131 are desirably arranged so as not to overlap with the piezoelectric material thin films 115 of the ultrasonic wave receiving elements 132. The transmission wave from the ultrasonic wave transmitting element 131 can be efficiently transmitted to the second surface 100b side, and when the transmission acoustic wave reaches the cavities 108a, an irregular reflection occurs to deteriorate reverberation characteristics, which is to be avoided.

Furthermore, in the ultrasonic wave receiving elements 132 constituted by the MEMS, an amplifier circuit or the like can be mounted on the same substrate, and it is possible to obtain the received signal having a high S/N ratio. On the other hand, it is difficult to realize a high transmission sound pressure in the MEMS. By providing the ultrasonic wave transmitting element 131 which is not the MEMS and the ultrasonic wave receiving elements 132 which are the MEMSs in the ultrasonic apparatus 100, both a high transmit strength and high reception sensitivity can be realized.

Furthermore, in the ultrasonic element having a general array structure, in order to prevent an acoustic crosstalk, separation grooves are provided between the ultrasonic elements, but the ultrasonic wave transmitting element 131 is not provided with the separation grooves in the piezoelectric material layer 102. This is because, since the ultrasonic wave transmitting element 131 and the ultrasonic wave receiving elements 132 are separated in the ultrasonic apparatus 100, the acoustic crosstalk in terms of the reverberation does not pose a problem.

As a result, a Dice & Fill process for forming the separation grooves in manufacturing steps can be omitted, and lower costs and lower TAT (Turn Around Time) can be realized. In addition, it is possible to further suppress the acoustic crosstalk by using a low piezoelectric material Q value such as a porous piezoelectric material or a 1-3 composite material in the piezoelectric material layer 102, for example, and to suppress a sidelobe in the transmission beam.

Thus, the ultrasonic apparatus 100 has high mass productivity and high reliability since a bonding process or the like with a wiring board is unnecessary. Therefore, the ultrasonic apparatus 100 has a high transmission strength and high reception sensitivity, and it is possible to realize an ultrasonic apparatus excellent in the mass productivity and the reliability.

[Manufacturing method]

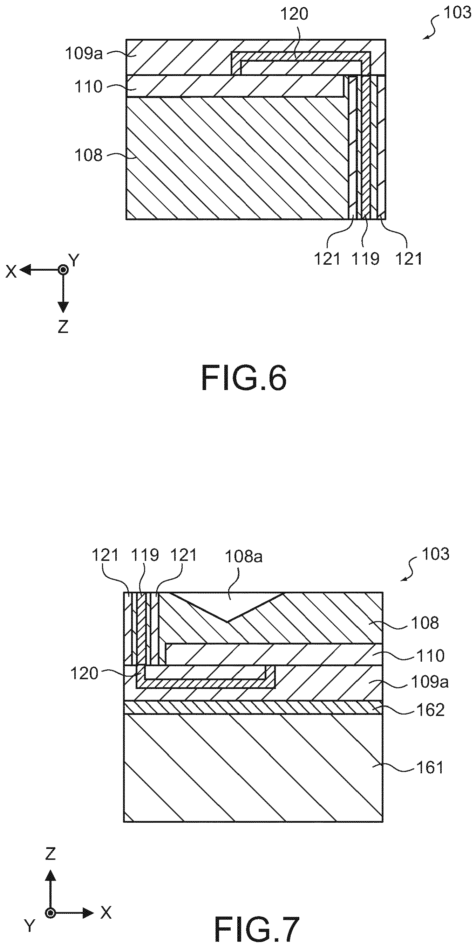

A method of manufacturing the ultrasonic apparatus 100 will be described. Figs. 6 to 14 are schematic diagrams showing manufacturing processes of the ultrasonic apparatus 100.

As shown in Fig. 6, the semiconductor circuit 110 is formed in the semiconductor substrate 103 having the semiconductor 108 and the insulator 109a. The semiconductor circuit 110 can be formed by a common Si-CMOS (Complementary metal-oxide-semiconductor field-effect transistor) process. At this time, the through electrode 119 and the insulator 121 are also formed.

Next, as shown in Fig. 7, the surface of the semiconductor substrate 103 on an insulator 109a side is bonded to the support substrate 161. The support substrate 161 is, for example, a quartz substrate. This bonding can be performed using a bonding layer 162 made of, for example, a UV curable resin.

Furthermore, the semiconductor 108 is thinned and patterned to form the cavity 108a. The processing can be carried out by dry etching such as Deep RIE (Reactive Ion Etching) or alkaline wet etching by using KOH, NaOH or TMAH (tetramethylammonium hydroxide).

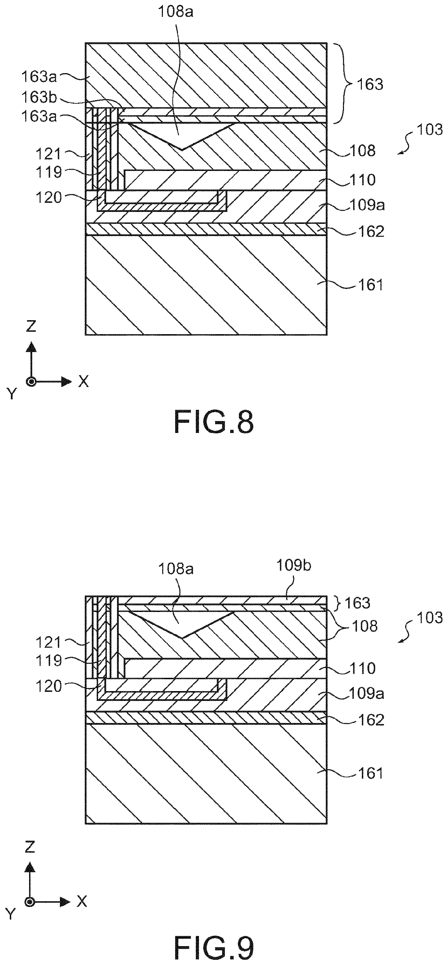

Subsequently, a SOI (Silicon on Insulator) substrate 163 is bonded to the semiconductor substrate 103 as shown in Fig. 8. The SOI substrate 163 includes a semiconductor 163a made of silicon and an insulator 163b made of silicon oxide, and the insulator 163b is a part to be the insulator 109b. On the SOI substrate 163, an alignment mark and an electrode corresponding to the through electrodes 119 are formed in advance.

Subsequently, as shown in Fig. 9, the SOI substrate 163 is thinned. As a result, a membrane structure of the ultrasonic wave receiving elements 132 is formed.

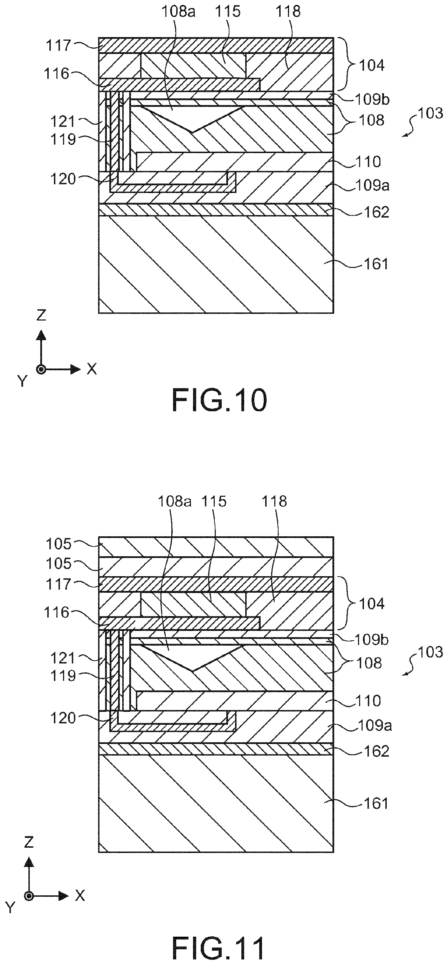

Subsequently, as shown in Fig. 10, the MEMS layer 104 is formed on the semiconductor substrate 103. The piezoelectric material thin films 115, the lower electrode films 116, the upper electrode film 117, and the interlayer insulating film 118 of the MEMS layer 104 can be formed by sputtering or CVD (chemical vapor deposition), and patterning by dry etching or the like.

In a case where the piezoelectric material thin films 115 are made of PZT, the lower electrode films 116 and the upper electrode film 117 are desirably formed of a material in which hardly undergo an interfacial reaction with the PZT such as Pt, Ti and ITO. The material of the piezoelectric material thin films 115 is not limited to the PZT, and may be sputtered films such as AlN.

Subsequently, as shown in Fig. 11, an acoustic matching layer 105 is formed on the MEMS layer 104. The acoustic matching layer 105 can be formed by spin coating or the like. The acoustic matching layer 105 may be blended with a filler to optimize acoustic impedance. In addition, when a matrix polymer such as a photosensitive solder resist that can be patterned by lithography facilitates opening of the electrode pads on the MEMS layer 104.

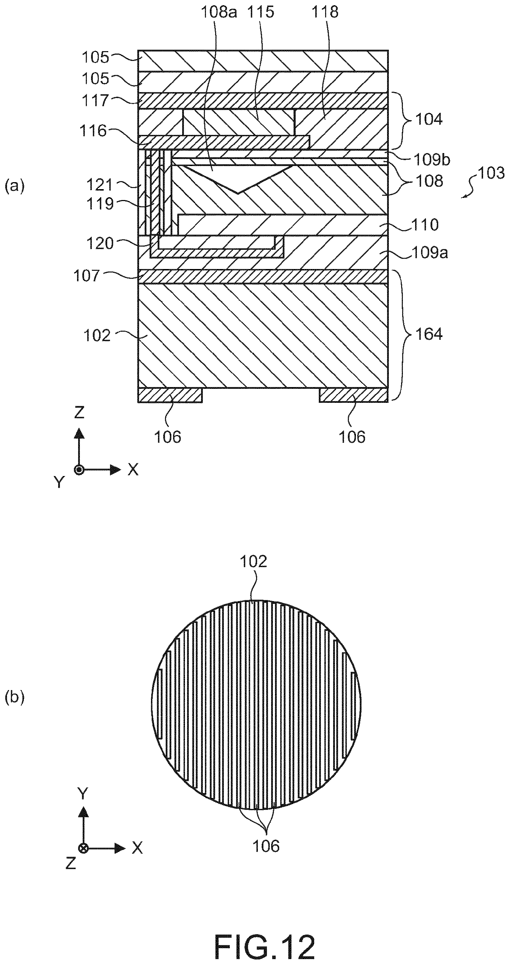

Subsequently, as shown in Fig. 12, the supporting substrate 161 and the bonding layer 162 are removed, and a transmission piezoelectric plate 164 is bonded to the semiconductor substrate 103. Fig. 12(a) is a cross-sectional diagram, and Fig. 12 (b) is a plan diagram as seen from a transmission piezoelectric plate 164 side. The transmission piezoelectric plate 164 is obtained by forming the lower electrodes 106 and the upper electrode 107 on the piezoelectric material layer 102. Fig. 12 is schematic diagram, the wiring width is, for example, 90 um wide, spacing of the wiring is 15μm, and a conventional semiconductor manufacturing technique is used.

As the piezoelectric material layer 102, for example, a single-piece disc article having a diameter of 150 mm and a thickness of 0.2 mm can be used. Metal films are formed as the lower electrodes 106 and the upper electrode 107 on the piezoelectric material layer 102 by a method such as PVD (physical vapor deposition), e.g., sputtering, plating or the CVD. The material of the metal films may be Ni, Ti, Au, Ag, Cu, or the like, but is not particularly limited as long as it is bonded to PZT.

Through-hole machining and electrode patterning are performed by dry etching on the piezoelectric material layer 102, which is bonded to the semiconductor substrate 103 by a substrate bonding technology. At this time, positions are aligned so that the electrode pads 112 pre-formed on the semiconductor substrate 103 (see Fig. 3) and the through via electrodes 111 are bonded.

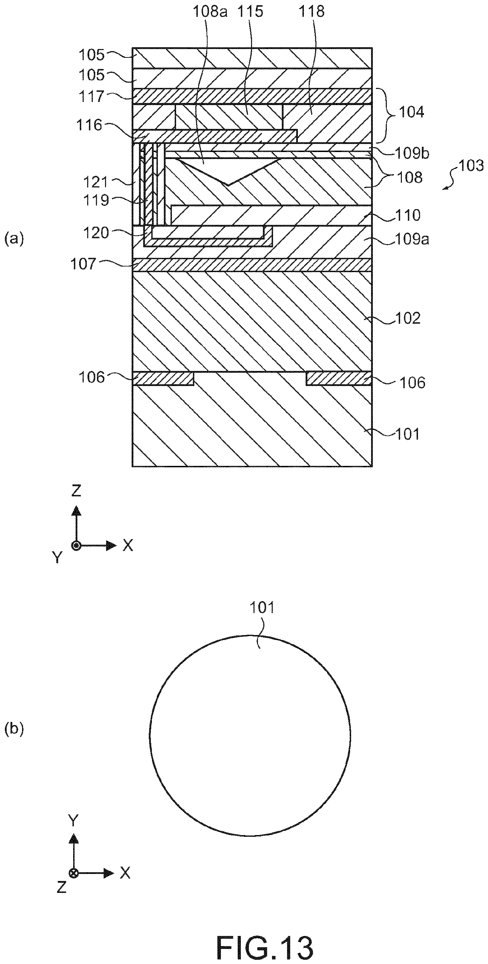

Subsequently, as shown in Fig. 13, the acoustic absorbing layer 101 is bonded to the transmission piezoelectric plate 164. Fig. 13(a) is a cross-sectional diagram, and Fig. 13(b) is a plan diagram seen from an acoustic absorbing layer 101 side.

The material of the acoustic absorbing layer 101 may be an acrylic or epoxy dispersed polymer used in a conventional backing absorbent material, or may be a material having a small acoustic impedance such as polyurethane. Polyurethane has the acoustic impedance of 1.5 MRayls, and the acoustic absorbing layer 101 can be functioned as a dematching structure in order to reduce the height.

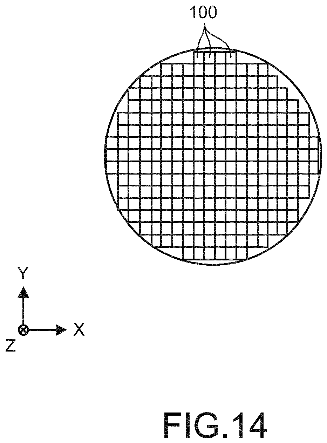

Finally, as shown in Fig. 14, a chip of the ultrasonic apparatus 100 is produced by cutting with a dicer. By employing the manufacturing processes as described above, the number of dicings can be greatly reduced as compared with the conventional Dice & Fill method, and the mass productivity can be improved. Furthermore, since it is possible to form a semiconductor circuit in the array region 151 (see Fig. 1), a mounting area can be significantly reduced.

[Semiconductors Circuit]

As described above, the semiconductor circuit 110 is provided on an opposite side of the MEMS layer 104 of the semiconductor substrate 103. The semiconductor circuit 110 is a current-voltage conversion circuit including a transimpedance amplifier and can convert a reception current output from the ultrasonic wave receiving elements 132 to a voltage. Furthermore, a signal amplifying circuit using a low-noise amplifier may be provided at the subsequent stage.
On the other hand, since a dynamic range of 50 to 80 dB or more is required for the sound pressure to be obtained by an ultrasonic measurement, providing a log amplifier (logarithmic conversion circuit) in the subsequent stage makes it possible to expand the dynamic range of the received signal to be handled.
Although the above circuits are not necessarily one-to-one correspondence to the respective ultrasonic wave receiving elements 132, it is possible to correspond one-to-one to the respective ultrasonic wave receiving elements 132 by arranging within footprints of the respective ultrasonic wave receiving elements 132. Thus, it is possible to amplify the output of each of the ultrasonic wave receiving elements 132 independently, and to also acquire an arrival time, phase difference information or the like of the ultrasonic wave reaching the ultrasonic wave receiving elements 132.
Furthermore, the semiconductor circuit 110 may include any one or more of an analog calculation circuit, an analog delay circuit, an analog-to-digital conversion circuit, and a digital calculation circuit. By these circuits, signal processing and image processing can be performed on the received signals of the ultrasonic wave receiving elements 132. By arranging these circuits on the semiconductor substrate 103, it is possible to achieve a significant miniaturization of the ultrasonic apparatus.
These circuits can also be selected for each ultrasonic wave receiving element 132 by an address selection. If an amount of data by one transmittance and reception is huge, the data is split. In a field in which a data rate is not required, there is also a method of completing scan data in a plurality of times of transmission and reception, such as synthetic aperture method in which the amount of data to be processed at a time is reduced and it is thus also possible to reduce an area of the semiconductor circuit.
These circuits may be arranged in the array region 151 (see Fig. 1) or may be arranged in the peripheral circuit region 152.
Furthermore, the driver circuit of the ultrasonic wave transmitting element 131 may be configured in the semiconductor circuit 110. As shown in Figs. 2 and 3, the semiconductor circuit 110 is provided with the driver circuit in the peripheral circuit region 152, and it is possible to connect the driver circuit and the lower electrodes 106 by the through via electrodes 111.
The driver circuit and the ultrasonic wave transmitting element 131 may be connected by wire bonding, a flexible substrate, an interposer or the like, but it is difficult in the semiconductor process. By connecting the driver circuit and the ultrasonic wave transmitting element 131 by the through via electrodes 111, it is possible to facilitate manufacturing.
Via processing to the piezoelectric material layer 102 for manufacturing the through via electrodes 111 can be performed by drilling or laser processing, or vias may be formed by arranging a mold pattern during firing.
As described above, the semiconductor circuit 110 may include either one or both of the signal processing circuit of the ultrasonic wave transmitting element 131 and the signal generating circuits of the ultrasonic wave receiving elements 132.
Specifically, the semiconductor circuit 110 may include one or more of the charge pumping circuit, the driver circuit, the address selection circuit, the amplifier circuit, the analog arithmetic circuit, the analog-to-digital conversion circuit, the digital arithmetic circuit, the power supply circuit, the modulation circuit, and the impedance matching circuit. Furthermore, the amplifier circuit may be any of a low noise circuit, a differential amplifier, and a logarithmic conversion amplifier.

Reference Signs List

| 100 | ultrasonic apparatus |
| 101 | acoustic absorbing layer |
| 102 | piezoelectric material layer |
| 103 | semiconductor substrate |
| 104 | MEMS layer |
| 105 | acoustic matching layer |
| 106 | lower electrode |
| 107 | upper electrode |
| 108 | semiconductor |
| 108a | cavity |

| 109a | insulator |
| --- | --- |
| 109b | insulator |
| 110 | semiconductor circuit |
| 111 | through via electrode |
| 115 | piezoelectric material thin film |
| 116 | lower electrode film |
| 117 | upper electrode film |
| 118 | interlayer insulating film |
| 119 | through electrode |
| 131 | ultrasonic wave transmitting element |
| 132 | ultrasonic wave receiving element |
| 151 | array region |
| 152 | peripheral circuit region |

**Claims**

1. An ultrasonic apparatus (100), comprising:

   an ultrasonic wave transmitting element (131) arranged on a first surface (100a) side of the ultrasonic apparatus and having a structure in which a single piezoelectric material layer (102) is sandwiched by a plurality of upper electrodes on one side and at least one lower electrode on the other side; and
   an ultrasonic wave receiving element (132) arranged between a second surface (100b) opposite to the first surface side of the ultrasonic apparatus and the ultrasonic wave transmitting element, wherein the ultrasonic wave receiving element has a MEMS (Micro Electro Mechanical Systems) structure,
   wherein the plurality of electrodes is for independently controlling the ultrasonic wave transmitting element as an array element.

2. The ultrasonic apparatus according to claim 1, wherein
   the piezoelectric material layer is not provided with a groove structure.

3. The ultrasonic apparatus according to claim 1 or claim 2, further comprising:
   an acoustic absorbing layer (101) made of an acoustic absorbing material and acoustically bonded to the ultrasonic wave transmitting element.

4. The ultrasonic apparatus according to claim 3, wherein
   the acoustic absorbing layer has an acoustic impedance equal to or less than 1/10 of an acoustic impedance of the piezoelectric material layer.

5. The ultrasonic apparatus according to any of claims 1 to 4, further comprising:
   an acoustic matching layer (105) provided on the ultrasonic wave receiving element on a side opposite to the ultrasonic wave transmitting element.

6. The ultrasonic apparatus according to any of claims 1 to 5, wherein
   the ultrasonic wave receiving element has a piezoelectric material thin film (115).

7. The ultrasonic apparatus according to claim 6, wherein
   the piezoelectric material thin film is a thin film having a thickness thinner than the piezoelectric material layer.

8. The ultrasonic apparatus according to any of claims 6 and 7, wherein
   the piezoelectric material thin film is made of an inorganic piezoelectric material, an organic piezoelectric material, or a combination thereof.

9. The ultrasonic apparatus according to any of claims 1 to 5, further comprising:
   a semiconductor substrate (103) arranged between the ultrasonic wave transmitting element and the ultrasonic wave receiving element.

10. The ultrasonic apparatus according to claim 9, wherein

a cavity is provided between the piezoelectric material thin film and the ultrasonic wave transmitting element in the semiconductor substrate.

11. The ultrasonic apparatus according to claim 9, wherein
a semiconductor circuit (110) is provided on a piezoelectric material layer side of the semiconductor substrate.

12. The ultrasonic apparatus according to claim 11, wherein
the semiconductor circuit includes one or more of a charge pumping circuit, a driver circuit, an address selection circuit, an amplifier circuit, an analog arithmetic circuit, an analog-to-digital conversion circuit, a digital arithmetic circuit, a power supply circuit, a modulation circuit, and an impedance matching circuit.

13. The ultrasonic apparatus according to any of claims 11 and 12, wherein
the semiconductor substrate has a through electrode (119) for connecting the ultrasonic wave receiving element and the semiconductor circuit.

14. The ultrasonic apparatus according to any of claims 11 and 12, wherein
the piezoelectric material layer has a through via electrode (111) for connecting the semiconductor circuit and the lower electrode.

**Patentansprüche**

1. Ultraschallvorrichtung (100), umfassend:

ein Ultraschallwellensendeelement (131), das auf einer ersten Oberflächenseite (100a) der Ultraschallvorrichtung angeordnet ist und eine Struktur aufweist, bei der eine einzelne Schicht (102) aus piezoelektrischem Material zwischen einer Vielzahl von oberen Elektroden auf einer Seite und mindestens einer unteren Elektrode auf der anderen Seite angeordnet ist; und
ein Ultraschallwellenempfangselement (132), das zwischen einer zweiten Oberfläche (100b), die der ersten Oberflächenseite der Ultraschallvorrichtung gegenüberliegt, und dem Ultraschallwellensendeelement angeordnet ist, wobei das Ultraschallwellenempfangselement eine "Micro Electro Mechanical System"-, MEMS-, Struktur aufweist, wobei die Vielzahl von Elektroden zur unabhängigen Steuerung des Ultraschallwellensendeelements als Arrayelement dient.

2. Ultraschallvorrichtung nach Anspruch 1, wobei die Schicht aus piezoelektrischem Material nicht mit einer Rillenstruktur bereitgestellt ist.

3. Ultraschallvorrichtung nach Anspruch 1 oder Anspruch 2, ferner umfassend:
eine schallabsorbierende Schicht (101), die aus einem schallabsorbierenden Material besteht und akustisch an das Ultraschallwellensendeelement gebunden ist.

4. Ultraschallvorrichtung nach Anspruch 3, wobei die schallabsorbierende Schicht eine akustische Impedanz aufweist, die gleich oder kleiner als 1/10 einer akustischen Impedanz der Schicht aus piezoelektrischem Material ist.

5. Ultraschallvorrichtung nach einem der Ansprüche 1 bis 4, ferner umfassend:
eine Akustikanpassungsschicht (105), die auf dem Ultraschallwellenempfangselement auf einer dem Ultraschallwellensendeelement gegenüberliegenden Seite bereitgestellt ist.

6. Ultraschallvorrichtung nach einem der Ansprüche 1 bis 5, wobei
das Ultraschallwellenempfangselement eine Dünnschicht (115) aus piezoelektrischem Material aufweist.

7. Ultraschallvorrichtung nach Anspruch 6, wobei
die Dünnschicht aus piezoelektrischem Material eine Dünnschicht mit einer geringeren Dicke als die Schicht aus piezoelektrischem Material ist.

8. Ultraschallvorrichtung nach einem der Ansprüche 6 und 7, wobei
die Dünnschicht aus piezoelektrischem Material aus einem anorganischen piezoelektrischen Material, einem organischen piezoelektrischen Material oder einer Kombination davon besteht.

**9.** Ultraschallvorrichtung nach einem der Ansprüche 1 bis 5, ferner umfassend:
ein Halbleitersubstrat (103), das zwischen dem Ultraschallwellensendeelement und dem Ultraschallwellenempfangselement angeordnet ist.

**10.** Ultraschallvorrichtung nach Anspruch 9, wobei
ein Hohlraum zwischen der Dünnschicht aus piezoelektrischem Material und dem Ultraschallwellensendeelement im Halbleitersubstrat bereitgestellt ist.

**11.** Ultraschallvorrichtung nach Anspruch 9, wobei
eine Halbleiterschaltung (110) auf einer Seite der Schicht aus piezoelektrischem Material des Halbleitersubstrats bereitgestellt ist.

**12.** Ultraschallvorrichtung nach Anspruch 11, wobei
die Halbleiterschaltung eines oder mehrere von einer Ladungspumpschaltung, einer Treiberschaltung, einer Adressauswahlschaltung, einer Verstärkerschaltung, einer analogen Rechenschaltung, einer Analog-Digital-Wandlerschaltung, einer digitalen Rechenschaltung, einer Stromversorgungsschaltung, einer Modulationsschaltung und einer Impedanzanpassungsschaltung beinhaltet.

**13.** Ultraschallvorrichtung nach einem der Ansprüche 11 und 12, wobei
das Halbleitersubstrat eine Durchgangselektrode (119) zum Verbinden des Ultraschallwellenempfangselements und der Halbleiterschaltung aufweist.

**14.** Ultraschallvorrichtung nach einem der Ansprüche 11 und 12, wobei
die Schicht aus piezoelektrischem Material eine Durchgangselektrode (111) zum Verbinden der Halbleiterschaltung und der unteren Elektrode aufweist.


**Revendications**

**1.** Appareil à ultrasons (100), comprenant :

un élément d'émission d'ondes ultrasonores (131) disposé sur le côté d'une première surface (100a) de l'appareil à ultrasons et ayant une structure dans laquelle une seule couche de matériau piézoélectrique (102) est prise en sandwich par une pluralité d'électrodes supérieures sur un côté et au moins une électrode inférieure sur l'autre côté ; et
un élément de réception d'ondes ultrasonores (132) disposé entre une deuxième surface (100b) à l'opposé du côté de la première surface de l'appareil à ultrasons et l'élément d'émission d'ondes ultrasonores, dans lequel l'élément de réception d'ondes ultrasonores a une structure MEMS (systèmes microélectromécaniques), dans lequel la pluralité d'électrodes sert à contrôler indépendamment l'élément d'émission d'ondes ultrasonores comme un élément d'un réseau.

**2.** Appareil à ultrasons selon la revendication 1, dans lequel
la couche de matériau piézoélectrique n'est pas pourvue d'une structure de rainure.

**3.** Appareil à ultrasons selon la revendication 1 ou la revendication 2, comprenant en outre :
une couche acoustique absorbante (101) constituée d'un matériau acoustique absorbant et reliée acoustiquement à l'élément d'émission d'ondes ultrasonores.

**4.** Appareil à ultrasons selon la revendication 3, dans lequel
la couche acoustique absorbante a une impédance acoustique égale ou inférieure à 1/10$^e$ d'une impédance acoustique de la couche de matériau piézoélectrique.

**5.** Appareil à ultrasons selon l'une quelconque des revendications 1 à 4, comprenant en outre :
une couche d'adaptation acoustique (105) disposée sur l'élément de réception d'ondes ultrasonores sur un côté opposé à l'élément d'émission d'ondes ultrasonores.

**6.** Appareil à ultrasons selon l'une quelconque des revendications 1 à 5, dans lequel
l'élément de réception d'ondes ultrasonores a un film mince de matériau piézoélectrique (115).

**7.** Appareil à ultrasons selon la revendication 6, dans lequel
le film mince de matériau piézoélectrique est un film mince ayant une épaisseur plus fine que la couche de matériau piézoélectrique.

**8.** Appareil à ultrasons selon l'une quelconque des revendications 6 et 7, dans lequel
le film mince de matériau piézoélectrique est constitué d'un matériau piézoélectrique inorganique, d'un matériau piézoélectrique organique, ou d'une combinaison de ceux-ci.

**9.** Appareil à ultrasons selon l'une quelconque des revendications 1 à 5, comprenant en outre :
un substrat semi-conducteur (103) disposé entre l'élément d'émission d'ondes ultrasonores et l'élément de réception d'ondes ultrasonores.

**10.** Appareil à ultrasons selon la revendication 9, dans lequel
une cavité est ménagée entre le film mince de matériau piézoélectrique et l'élément d'émission d'ondes ultrasonores dans le substrat semi-conducteur.

**11.** Appareil à ultrasons selon la revendication 9, dans lequel
un circuit semi-conducteur (110) est disposé sur un côté de la couche de matériau piézoélectrique du substrat semi-conducteur.

**12.** Appareil à ultrasons selon la revendication 11, dans lequel
le circuit semi-conducteur comporte un ou plusieurs éléments parmi un circuit de pompage de charge, un circuit de commande, un circuit de sélection d'adresse, un circuit amplificateur, un circuit arithmétique analogique, un circuit de conversion analogique-numérique, un circuit arithmétique numérique, un circuit d'alimentation électrique, un circuit de modulation, et un circuit d'adaptation d'impédance.

**13.** Appareil à ultrasons selon l'une quelconque des revendications 11 et 12, dans lequel
le substrat semi-conducteur a une électrode traversante (119) destinée à connecter l'élément de réception d'ondes ultrasonores et le circuit semi-conducteur.

**14.** Appareil à ultrasons selon l'une quelconque des revendications 11 et 12, dans lequel
la couche de matériau piézoélectrique a une électrode d'interconnexion verticale (111) destinée à connecter le circuit semi-conducteur et l'électrode inférieure.

FIG.1

FIG.2

FIG.3

EP 3 905 716 B1

FIG.4

FIG.5

FIG.6

FIG.7

FIG.8

FIG.9

FIG.10

FIG.11

FIG.12

(a)

(b)

FIG.13

FIG.14

**EP 3 905 716 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011071842 A

- DE 2012002438 T5